# EUROPEAN PATENT APPLICATION

(11) **EP 1 199 074 A1**
(43) Date of publication of application: **24.04.2002**
(21) Application number: 01402325.3
(22) Date of filing: 10.09.2001
(51) Int. Cl.: A61K 31/277, A61K 31/4015, A61K 31/00, A61K 31/5517, A61K 31/551, A61K 31/519, A61P 37/00, A61P 37/08, A61P 39/00, A61P 17/00, A61P 1/00, A61P 1/16, A61P 3/10, A61P 9/10, A61P 11/00, A61P 19/02, A61P 17/06, A61P 35/02, A61P 37/06, A61P 25/00

(54) **Pharmaceutical composition for preventing or treating a disease associated with an excess of il-12 production**

(30) Priority: 15.09.2000 EP 00402560
(71) Applicant: WARNER-LAMBERT COMPANY, Morris Plains New Jersey 07950 (US)
(72) Inventor: Moulon, Corinne, 1066 Epalinges (CH); Heysteck, Heleen, 3605 HE Maarssen (NL)
(74) Representative: Dufresne, Guillaume Alain François

(57) **Abstract**

This invention relates to the use of a PDE4 inhibitor or a pharmacologically acceptable salt thereof for manufacturing a medicament for preventing or treating a disease associated with an excess in IL-12 production.

## Description

### FIELD OF THE INVENTION

This invention relates to the use of a PDE4 inhibitor or a pharmacologically acceptable salt thereof for manufacturing a medicament for preventing or treating a disease associated with an excess in IL-12 production.

### BACKGROUND OF THE INVENTION

Dendritic cells are professional antigen-presenting cells (APC) that play a crucial role in the immune system because they have the unique ability to activate naive T-cells. Dendritic cells produce IL-12p70 upon interaction between CD40 on the dendritic cell and CD40L on the activated T helper lymphocyte. IL-12 plays a central role by inducing γ-Interferon (IFN-γ) production by T-cells and natural killer (NK) cells, enhancing NK cell cytotoxicity and promoting the development of cytotoxic T-cells.

Interleukin-12 (IL-12) is a cytokine produced by myelomonocytic cells as a hetero-dimer composed of two disulphide-linked chains, p35 and p40, encoded by separate chains. Simultaneous expression of the two genes is required for the production of the biologically active IL-12p70 heterodimer.

In addition, IL-12 has been shown to direct immune response towards T-helper 1 (Th1) type responses which are characterized by high interferon-γ and low IL-4 production. Because an excess of Th1-type cells occurs in various pathologies, typically auto-immune diseases, multiple sclerosis, diabetes, arthritis and also auto-immune demyelinating disorders, there is a need in the art for compounds having the ability to inhibit IL-12 production within the context of these pathologies.

Some molecules have been shown in the art to inhibit IL-12 production by dendritic cells, such as salbutamol, a β₂-agonist (Panina-Bordignon P. et al., 1997, J. Clin. Invest., vol.100 (6): 1513-1519), prostaglandin E₂ (Kalinski P. et al., 1997 et al.,1997, The Journal of Immunology, 159(1): 28-35) and cholera toxin (Braun M.C. et al., 1999 , The Journal of Experimental Medicine, vol.189 (3):541-552.). However, these molecules, particularly cholera toxin and prostaglandin E₂, cannot be used safely for treating patients.

There is thus a need for drugs that can act on disorders wherein the production of IL-12 is involved, such as auto-immune disorders.

### SUMMARY OF THE INVENTION

The invention is directed to the use of a PDE4 inhibitor for manufacturing a medicament for preventing and/or treating a disease associated with an excess in IL-12 production as well as to a method for treating such disorders through the use of at least one PDE4 inhibitor.

It is also related to a pharmaceutical composition for preventing and/or treating a disease associated with an excess in IL-12 production comprising an effective amount of at least one PDE4 inhibitor.

### BRIEF DESCRIPTION OF THE FIGURES

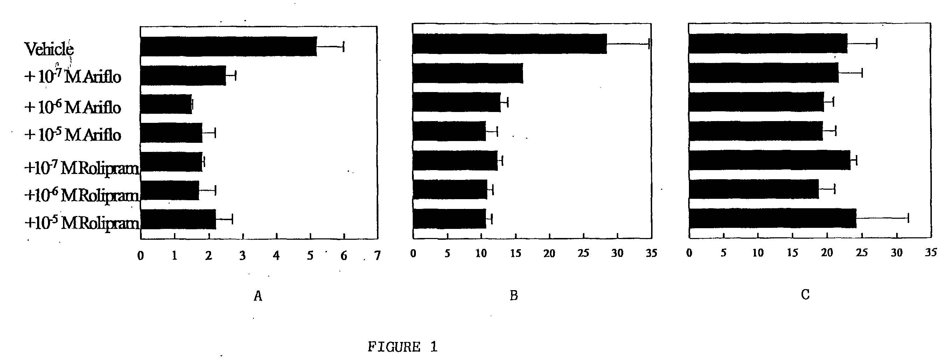

**Figure 1** illustrates the inhibitory effect of PDE 4 inhibitors on LPS plus IFN-γ-induced cytokine production by immature DC. DC (2 x 10⁴ cells/well) were stimulated with LPS and IFN-γ combined with the indicated concentrations of PDE4 inhibitors (Ariflo or Rolipram). Results are expressed as the mean cytokine production in ng/ml of duplicate cultures (24h supernatants). A: IL-12p70 ; B : TNF-α; C: IL-6. Cytokine production is expressed as ng/ml.

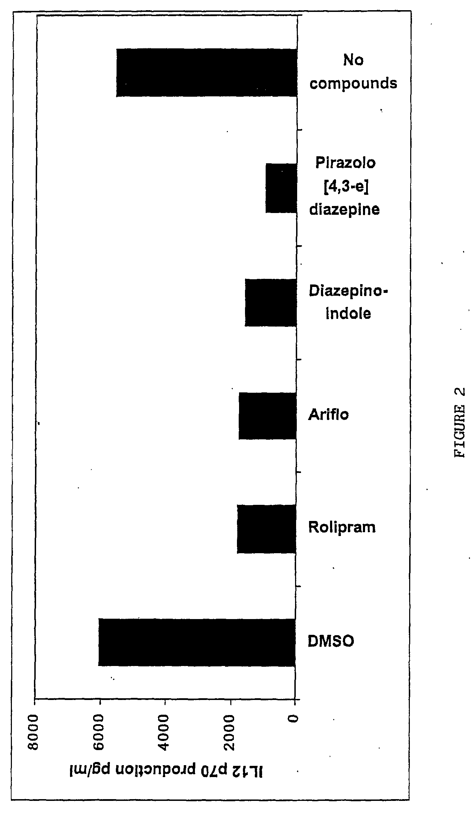

**Figure 2** illustrates the inhibitory effect of PDE4 inhibitors on CD40-induced IL12 production by immature DC. DC (2x10⁴ cells/well) were stimulated with J558-CD40L cells in the presence of 10µM of the indicated PDE4 inhibitors (Ariflo, Rolipram, Diazepino-indole or Pirazolo [4,3-e] diazepine). Results are expressed as the cytokine production in pg/ml of 24h supernatant of cell cultures. The data shown are from one representative experiment out of three giving similar results.

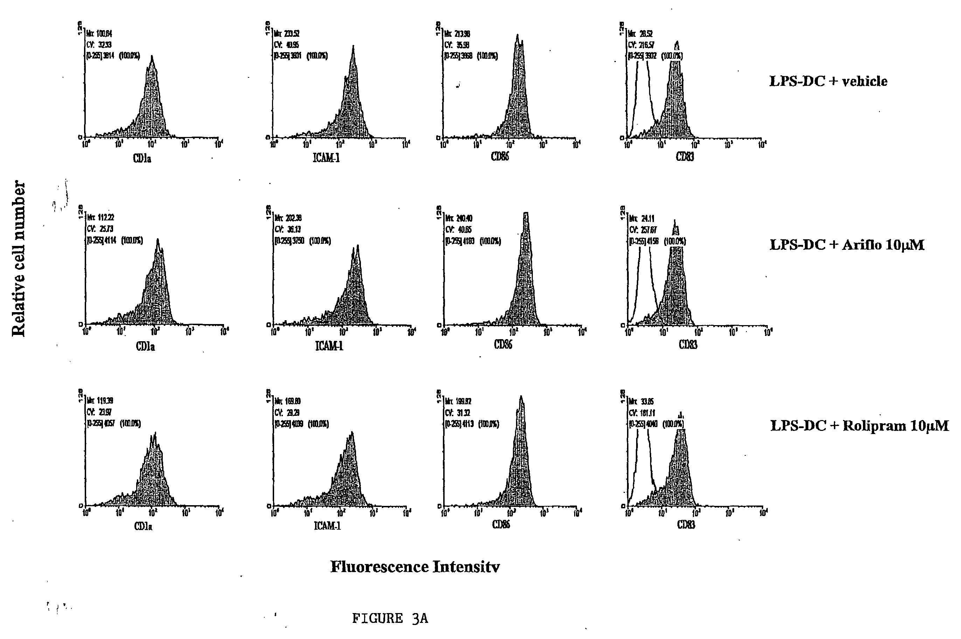

**Figure 3A** illustrates the absence of phenotypic effect of the presence of PDE4 inhibitors during maturation of dendritic cells. Dendritic cells were matured for 2 days with LPS in the presence or absence of the PDE4 inhibitors Ariflo or Rolipram and analysed for the expression of cell surface molecules by flow cytometry. In the left corner of each figure the mean fluorescence intensity (Mn).

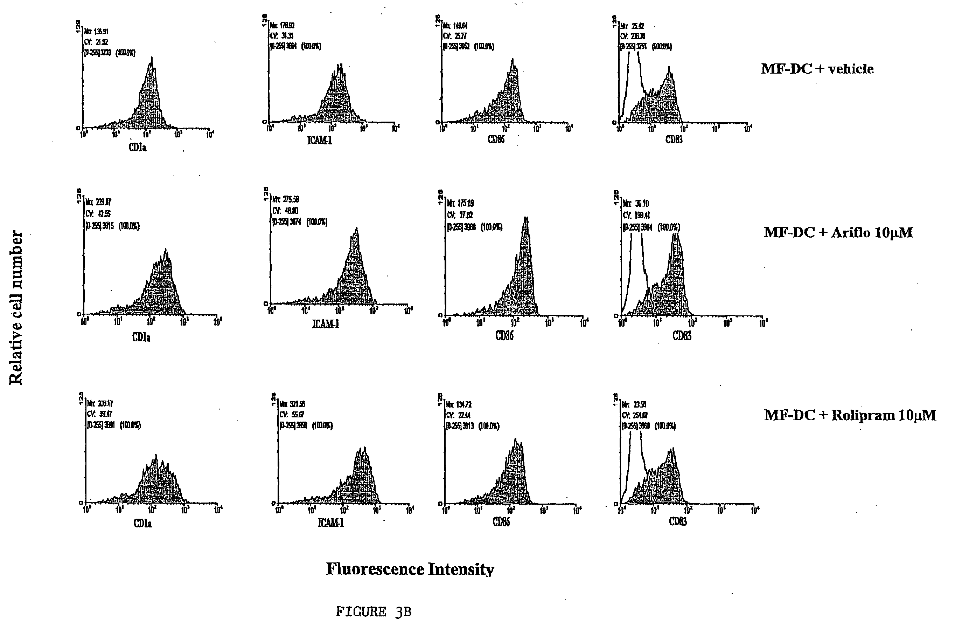

**Figure 3B** illustrates the absence of phenotypic effect of the presence of PDE4 inhibitors during maturation of dendritic cells. Dendritic cells were matured for 2 days with the maturation factors TNF-α plus IL-1β (referred to as MF) in the presence or absence of the PDE4 inhibitors Ariflo and Rolipram and analysed for the expression of cell surface molecules by flow cytometry. In the left corner of each figure the mean fluorescence intensity (Mn).

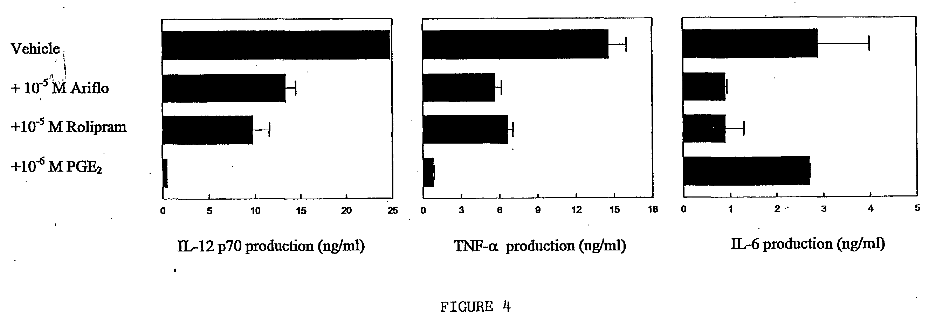

**Figure 4** illustrates the effect of PDE4 on cytokine production by mature dendritic cells. Maturation of dendritic cells was induced by IL-1β/TNF-α in either the absence or presence of PDE4 inhibitors, or PGE₂. Differently matured dendritic cells were harvested after 48 h, washed to remove residual factors, and stimulated with J558-CD40L and IFN-γ. Cytokine concentrations in 24 h supernatants were determined by ELISA. Results are expressed as the mean cytokine production in ng/ml +/- SD of duplicate cultures.

### DETAILED DESCRIPTION OF THE INVENTION

PDE4 inhibitors are known in the art to possess direct activities on T-cells, mainly by inhibiting T-cell proliferation, notably by downregulating antigen-driven proliferation.

The inventors have surprisingly found that PDE4 inhibitors exert a direct effect on dendritic cells by inhibiting their capacity to produce the inflammatory cytokine IL-12 upon subsequent stimulation of these dendritic cells, for example by a combination of LPS and IFN-γ or CD40L, a surface receptor of T-cell. They have also shown that although PDE4 inhibitors are able to inhibit IL-12 production by stimulated dendritic cells, these inhibitors do not alter the phenotype of mature dendritic cells.

Further, it has been shown according to the invention that the direct activity of PDE4 inhibitors on dendritic cells was able to induce changes in the T-helper cell population. More specifically, the direct activity of PDE4 inhibitors on dendritic cells direct the immune response of naive T cells towards a Th2-type response upon activation by mature dendritic cells pretreated by a PDE4 inhibitor during maturation. In addition, the direct activity of PDE4 inhibitors on dendritic cells induces a significant decrease of the Th1 population.

A first object of the invention consists of the use of at least one PDE4 inhibitor or a pharmaceutically acceptable salt thereof for manufacturing a medicament for preventing and/or treating a disease associated with an excess in IL-12 production.

The term "PDE4 inhibitor" is intended to encompass every compound which possesses the ability to inhibit a PDE4 enzyme with an IC₅₀ value of at least the same order of magnitude than the IC₅₀ measured in the same experimental condition for Rolipram. The IC₅₀ value corresponds to the molar concentration of the PDE4 inhibitor which is able to inhibit 50% of the PDE4 activity as regards the value of PDE4 activity measured with control buffer in the absence of PDE4 inhibitor.

As an illustrative embodiment, PDE4 inhibition may be measured by the one skilled in the art according to the method described by TORPHY et al. (1992, J.Pharm. Exp. Ther. Vol. 263: 1195-1205).

Most preferably, the determination of the IC₅₀ value for a particular PDE4 inhibitor is achieved by measuring the PDE4 enzyme activity both in the absence of any inhibitor and in the presence of a PDE4 inhibitor in a range of inhibitor concentrations comprised between 0.01 nM and 100 µM and preferably between 0.01 nM and 1 µM.

PDE4 enzyme which is used for the inhibition test may be obtained from a cytosolic extract from cells of the human cell line U937.

Without wishing to be bound by any particular theory, the inventors believe that the direct activity of PDE4 inhibitors on IL-12 production by dendritic cells is not, at least only, mediated by a raise in intracellular cAMP concentration. Indeed, it is shown that other PDE inhibitors, which are known to increase cAMP levels like PDE7 inhibitors, do not exert any inhibiting activity on IL-12 production by dendritic cells.

The expression " an excess in IL-12 production " is intended herein to encompass an undesirable production of IL-12 in physiological situations wherein the development of an immune response in a patient body causes a strong inflammatory reaction or a high activation of T-helper cells which is detrimental for the health of said patient, leading to various pathologies including auto-immune diseases.

Diseases associated with an excess in IL-12 production encompass auto-immune disorders like Inflammatory of the bronchi/pathologies affecting the bronchus such as Bronchoconstriction, multiple organ failure, Osteoarthritis, Septic shock (septicaemia), Inflammatory complaints or disorders, (Chronic) inflammatory diseases of the digestive system including intestine (hemorrhagic rectocolitis, ulcerative colitis, IBD) with auto-immune component, chronic lymphatic leukaemia, Hepatic failure including following immunologic or inflammatory liver injury, Graft rejection, Crohn's disease, Chronic obstructive bronchopneumopathy (chronic bronchitis, emphysema, COPD), acute pulmonary attack, ischemia-induced neuronal damages, Reperfusion ischaemia diseases related to a high level of TNF-α, acute respiratory distress syndrome, acute pancreatitis, auto-immune demyelinating disorders, multiple sclerosis, Type I diabetes, chronic/relapsing allergic encephalomyelitis, auto-immune encephalomyelitis, inflammatory colitis, arthritis including rheumatoid arthritis, sarcoidiosis, hypersensitivity pneumonitis, auto-immune uveitis, Inflammatory Bowel Disease (IBD), psoriasis, eczema, contact dermatitis, as well as other Th1-mediated human diseases.

In a first preferred embodiment, the PDE4 inhibitor is selected from the group consisting of Rolipram and Ariflo.

In a second preferred embodiment, the PDE4 inhibitor is selected from the group consisting of compounds from the Diazepino-indole family, preferably [1,4]diazepino[6,7,1-*hi*] indoles such as those disclosed in the PCT applications WO 97/36905 and WO 96/11690 and as well as their metabolites disclosed in the PCT Application N° WO 99/50270.

Preferred [1,4]diazepino[6,7,1-*hi*]indoles compounds are the following compounds :
- (3R)-isoquinoline-3-carboxylic acid (9-hydroxy-4-oxo-1-phenyl-3,4,6,7-tetrahydro[1,4]diazepino[6,7,1-hi]indol-3-yl)amide
- (3R)-4-t-butyloxycarbonylamino-N-(9-amino-4-oxo-1-phenyl-3,4,6,7-tetrahydro[1,4]diazepino[6,7,1-hi]indol-3-yl)benzamide
- (3R)-4-amino-N-(9-amino-4-oxo-1-phenyl-3,4,6,7-tetrahydro[1,4] diazepino [6,7,1-hi]indol-3-yl)-3,5-dichlorobenzamide
- (3R)-4-amino-N-(9-amino-4-oxo-1-phenyl-3,4,6,7-tetrahydro[1,4]diazepino [6,7,1-hi]indol-3-yl)-5-chloro-2-methoxybenzamide
- (3R)-N-(9-amino-4-oxo-1-phenyl-3,4,6,7-tetrahydro-[1,4]diazepino[6,7,1-hi]indol-3-yl)isonicotinamide
- (3R)-3-t-butyloxycarbonylamino-N-(9-amino-4-oxo-1-phenyl-3,4,6,7-tetrahydro[1,4]diazepino[6,7,1-hi]indol-3-yl)isonicotinamide
- (3R)-isoquinoline-3-carboxylic acid (9-amino-4-oxo-1-phenyl-3,4,6,7-tetrahydro[1,4]diazepino[6,7,1-hi]indol-3-yl)amide
- (3R)-quinoline-3-carboxylic acid (9-amino-4-oxo-1-phenyl-3,4,6,7-tetrahydro[1,4]diazepino[6,7,1-hi]indol-3-yl)amide
- (3R)-4,7-dimethylpyrazolo[5,1-c][1,2,4]triazine-3-carboxylic acid (9-amino-4-oxo-1-phenyl-3,4,6,7-tetrahydro[1,4]diazepino[6,7,1-hi]indol-3-yl)amide,
- (3R)-4-amino-3,5-dichloro-N-(9-dimethylamino-4-oxo-1-phenyl-3,4,6,7-tetrahydro[1,4]diazepino[6,7,1-hi]indol-3-yl)benzamide,
- (3R)N-(9-amino-4-oxo-1-phenyl-3,4,6,7-tetrahydro-[1,4]diazepino[6,7,1-*hi*]indol-3-yl)-2-benzofuranecarboxamide,
   (3R)4,7-dimethyl-pyrazolo[5,1-c][1,2,4]triazine-3-carboxylic acid[4-oxo-1-phenyl-9-(pyrrolidin-1-yl)-3,4,6,7-tetrahydro-[1,4]diazepino[6,7,1-*hi*]indol-3-yl]-amide.

Among the compounds disclosed in the PCT Application N° PCT/FR00/01174 (WO 00/66584), 1-aminotriazolo[4,3-a]quinazolin-5-ones and/or -5-thiones are preferred and more particularly, the following compounds:
1-(Azepan-1-yl)-7-chloro-4-(3-phenylallyl)-4H-[1,2,4]triazolo[4,3-a]quinazolin-5-one
7-Bromo-1-dimethylamino-4-((E)-3-pyrid-3-ylallyl)-4H-[1,2,4]triazolo[4,3-a] quinazolin-5-one1-(azepan-1-yl)-7-chloro-4-(4-chlorobenzyl)-4H-[1,2,4]triazolo[4,3-a]quinazolin-5-one
1-(azepan-1-yl)-7-chloro-4-(4-fluorobenzyl)-4H-[1,2,4]triazolo[4,3-a]quinazolin-5-one
1-(azepan-1-yl)-7-chloro-4-(3,4-dimethoxybenzyl)-4H-[1,2,4]triazolo[4,3-a]quinazolin-5-one
1-(azepan-1-yl)-7-chloro-4-(3-pyridylmethyl)-4H-[1,2,4]triazolo[4,3-a]quinazolin-5-one
1-(azepan-1-yl)-7-bromo-4-(4-chlorophenylmethyl)-4H-[1,2,4]triazolo[4,3-a]quinazolin-5-one
4-(1-Azepan-1-yl-7-bromo-5-oxo-5H-[1,2,4]triazolo[4,3-a]quinazolin-4-ylmethyl)benzonitrile
1-Azepan-1-yl-7-bromo-4-(3,4-dimethoxybenzyl)-4H-[1,2,4]triazolo[4,3-a]quinazolin-5-one
1-(azepan-1-yl)-7-bromo-4-(3-phenylallyl)-4H-[1,2,4]triazolo[4,3-a]quinazolin-5-one
1-Azepan-1-yl-7-bromo-4-((E)-3-pyrid-3-ylallyl)-4H-[1,2,4]triazolo[4,3-a]quinazolin-5-one
1-Azepan-1-yl-7-bromo-4-(3-pyrid-4-ylallyl)-4H-[1,2,4]triazolo[4,3-a]quinazolin-5-one
7-Bromo-4-(4-methylbenzyl)-1-pyrrolidin-1-yl-4H-[1,2,4]triazolo[4,3-a]quinazolin-5-one
7-Bromo-4-(4-chlorobenzyl)-1-pyrrolidin-1-yl-4H-[1,2,4]triazolo[4,3-a]quinazolin-5-one
7-Bromo-4-(4-fluorobenzyl)-1-pyrrolidin-1-yl-4H-[1,2,4]triazolo[4,3-a]quinazolin-5-one
4-(7-Bromo-5-oxo-1-pyrrolidin-1-yl-5H-[1,2,4]triazolo[4,3-a]quinazolin-4-ylmethyl)benzonitrile
4-(7-Bromo-5-oxo-1-pyrrolidin-1-yl-5H-[1,2,4]triazolo[4,3-a]quinazolin-4-ylmethyl)benzoic acid methyl ester
7-Bromo-4-(4-nitrobenzyl)-1-pyrrolidin-1-yl-4H-[1,2,4]triazolo[4,3-a]quinazolin-5-one
7-Bromo-4-(4-methoxybenzyl)-1-pyrrolidin-1-yl-4H-[1,2,4]triazolo[4,3-a]quinazolin-5-one
Acetic acid 4-(7-bromo-5-oxo-1-pyrrolidin-1-yl-5H-[1,2,4]triazolo[4,3-a]quinazolin-4-ylmethyl)phenyl ester
7-Bromo-4-(4-hydroxybenzyl)-1-pyrrolidin-1-yl-4H-[1,2,4]triazolo[4,3-a]quinazolin-5-one
7-Bromo-4-(3,4-dimethoxybenzyl)-1-pyrrolidin-1-yl-4H-[1,2,4]triazolo[4,3-a]quinazolin-5-one
1-(pyrrolidin-1-yl)-7-bromo-4-(3-phenylallyl)-4H-[1,2,4]triazolo[4,3-a]quinazolin-5-one
7-Bromo-4-[(E)-3-(4-chlorophenyl)allyl]-1-pyrrolidin-1-yl-4H-[1,2,4]triazolo[4,3-a]quinazolin-5-one
7-Bromo-4-[3-(4-methoxyphenyl)allyl]-1-pyrrolidin-1-yl-4H-[1,2,4]triazolo[4,3-a]quinazolin-5-one
7-Bromo-4-(3-pyrid-3-ylallyl)-1-pyrrolidin-1-yl-4H-[1,2,4]triazolo[4,3-a]quinazolin-5-one
7-Bromo-4-((E)-3-pyrid-4-ylallyl)-1-pyrrolidin-1-yl-4H-[1,2,4]triazolo[4,3-a]quinazolin-5-one
7-Bromo-4-(3,4-dimethoxybenzyl)-1-piperid-1-yl-4H-[1,2,4]triazolo[4,3-a]quinazolin-5-one
1-(piperid-1-yl)-7-bromo-4-(3-phenylallyl)-4H-[1,2,4]triazolo[4,3-a]quinazolin-5-one
7-Bromo-1-dimethylamino-4-(4-methylbenzyl)-4H-[1,2,4]triazolo[4,3-a]quinazolin-5-one
4-(7-Bromo-1-dimethylamino-5-oxo-5H-[1,2,4]triazolo[4,3-a]quinazolin-4-ylmethyl)benzonitrile
7-Bromo-1-dimethylamino-4-(4-hydroxybenzyl)-4H-[1,2,4]triazolo[4,3-a]quinazolin-5-one
4-(7-Bromo-1-dimethylamino-5-oxo-5H-[1,2,4]triazolo[4,3-a]quinazolin-4-ylmethyl)benzoic acid methyl ester
[4-(7-Bromo-1-dimethylamino-5-oxo-5H-[1,2,4]triazolo[4,3-a]quinazolin-4-ylmethyl)phenyl]acetonitrile
7-Bromo-1-dimethylamino-4-(3-phenylallyl)-4H-[1,2,4]triazolo[4,3-a]quinazolin-5-one
7-Bromo-1-dimethylamino-4-(3-phenyl-prop-2-ynyl)-4H-[1,2,4]triazolo[4,3-a]quinazolin-5-one
1-Azepan-1-yl-7-methyl-4-(3-phenylallyl)-4H-[1,2,4]triazolo[4,3-a]quinazolin-5-one
4-(3,4-Dimethoxybenzyl)-7-methyl-1 -pyrrolidin-1-yl-4H-[1,2,4]triazolo[4,3-a]quinazolin-5-one
[4-(7-Methyl-5-oxo-1-pyrrol idin-1-yl-5H-[1,2,4]triazolo[4,3-a]qu inazolin-4-ylmethyl)phenyl]acetic acid
7-Methyl-4-(3-phenylallyl)-1-pyrrolidin-1-yl-4H-[1,2,4]triazolo[4,3-a]quinazolin-5-one
[4-(1-Dimethylamino-7-methyl-5-oxo-5H-[1,2,4]triazolo[4,3-a]quinazolin-4-ylmethyl)phenyl]acetic acid
1-Dimethylamino-7-methyl-4-((E)-3-phenylallyl)-4H-[1,2,4]triazolo[4,3-a]quinazolin-5-one
1-Dimethylamino-7-methyl-4-(3-pyrid-3-ylallyl)-4H-[1,2,4]triazolo[4,3-a]quinazolin-5-one
[4-(7-Bromo-5-oxo-1-perhydroazepin-1-yl-5H-[1,2,4]triazolo[4,3-a]quinazolin-3-ylmethyl)phenyl]acetic acid
4-benzyl-7-bromo-1-(pyrrolidin-1-yl)-4H-[1,2,4]triazolo[4,3-a]quinazolin-5-one
4-Benzyl-7-bromo-1-(2, 5-dihydropyrrol-1-yl)-4H-[1,2,4]triazolo[4,3-a]quinazolin-5-one
4-Benzyl-7-iodo-1-pyrrolidin-1-yl-4H-[1,2,4]triazolo[4,3-a]quinazolin-5-one
1-Azepan-1-yl-4-benzyl-7-methyl-4H-[1,2,4]triazolo[4,3-a]quinazolin-5-one
4-(4-Aminobenzyl)-7-bromo-1-pyrrolidin-1-yl-4H-[1,2,4]triazolo[4,3-a]quinazolin-5-one
7-Amino-4-((E)-3-phenylallyl)-1-pyrrolidin-1-yl-4H-[1,2,4]triazolo[4,3-a]quinazolin-5-one
7-Amino-1-azepan-1-yl-4-benzyl-4H-[1,2,4]triazolo[4,3-a]quinazolin-5-one
7-Amino-4-((E)-3-pyrid-3-ylallyl)-1-pyrrolid in-1-yl-4H-[1,2,4]triazolo[4,3-a]quinazolin-5-one
7-Amino-1-dimethylamino-4-((E)-3-phenylallyl)-4H-[1,2,4]triazolo[4,3-a]quinazolin-5-one
4-Benzyl-7-methylamino-1-pyrrolidin-1-yl-4H-[1,2,4]triazolo[4,3-a]quinazolin-5-one
4-(7-Methylamino-5-oxo-1-pyrrolid in-1-yl-5H-[1,2,4]triazolo[4,3-a]quinazolin-4-ylmethyl)benzonitrile
4-Benzyl-8-methylamino-1-pyrrolidin-1-yl-4H-[1,2,4]triazolo[4,3-a]quinazolin-5-one
4-Benzyl-7-ethylamino-1-pyrrolidin-1-yl-4H-[1,2,4]triazolo[4,3-a]quinazolin-5-one
4-Benzyl-7-isopropylamino-1-pyrrolidin-1-yl-4H-[1,2,4]triazolo[4,3-a]quinazolin-5-one
[4-(7-Bromo-5-oxo-1-pyrrolidin-1-yl-5H-[1,2,4]triazolo[4,3-a]quinazolin-4-ylmethyl)phenyl]acetic acid methyl ester
2-[4-(7-Bromo-5-oxo-1-pyrrolidin-1-yl-5H-[1,2,4]triazolo[4,3-a]quinazolin-4-ylmethyl)phenyl]-N-methylacetamide
2-[4-(7-Bromo-1-dimethylamino-5-oxo-5H-[1,2,4]triazolo[4,3-a]quinazolin-4-ylmethyl)phenyl]acetamide
2-[4-(7-Bromo-5-oxo-1-pyrrolidin-1 -yl-5H-[1,2,4]triazolo[4,3-a]quinazolin-4-ylmethyl)phenyl]-N,N-dimethylacetamide
2-[4-(7-Bromo-5-oxo-1-pyrrolidin-1-yl-5H-[1,2,4]triazolo[4,3-a]qu inazolin-4-ylmethyl)phenyl]-N-hydroxyacetamide
1-Dimethylamino-7-methyl-4-(3-pyrid-3-ylallyl)-4H-[1,2,4]triazolo[4,3-a]quinazoline-5-thione

Among the compounds mentioned above, the following compounds are particularly preferred :
7-Bromo-1-dimethylamino-4-((E)-3-pyrid-3-ylallyl)-4H-[1,2,4]triazolo[4,3-a]quinazolin-5-one
1-(azepan-1-yl)-7-chloro-4-(3,4-dimethoxybenzyl)-4H-[1,2,4]triazolo[4,3-a]quinazolin-5-one
1-(azepan-1-yl)-7-chloro-4-(3-pyridylmethyl)-4H-[1,2,4]triazolo[4,3-a]quinazolin-5-one
4-(1-Azepan-1-yl-7-bromo-5-oxo-5H-[1,2,4]triazolo[4,3-a]quinazolin-4-ylmethyl)benzonitrile
1-Azepan-1-yl-7-bromo-4-(3,4-dimethoxybenzyl)-4H-[1,2,4]triazolo[4,3-a]quinazolin-5-one
1-(azepan-1-yl)-7-bromo-4-(3-phenylallyl)-4H-[1,2,4]triazolo[4,3-a]quinazolin-5-one
1-Azepan-1-yl-7-bromo-4-((E)-3-pyrid-3-ylallyl)-4H-[1,2,4]triazolo[4,3-a]quinazolin-5-one
1-Azepan-1-yl-7-bromo-4-(3-pyrid-4-ylallyl)-4H-[1,2,4]triazolo[4,3-a]quinazolin-5-one
7-Bromo-4-(4-methylbenzyl)-1-pyrrolidin-1-yl-4H-[1,2,4]triazolo[4,3-a]quinazolin-5-one
7-Bromo-4-(4-chlorobenzyl)-1-pyrrolidin-1-yl-4H-[1,2,4]triazolo[4,3-a]quinazolin-5-one
7-Bromo-4-(3,4-dimethoxybenzyl)-1-pyrrolidin-1-yl-4H-[1,2,4]triazolo[4,3-a]quinazolin-5-one
1-(pyrrolidin-1-yl)-7-bromo-4-(3-phenylallyl)-4H-[1,2,4]triazolo[4,3-a]quinazolin-5-one
7-Bromo-4-(3-pyrid-3-ylallyl)-1-pyrrolidin-1-yl-4H-[1,2,4]triazolo[4,3-a]quinazolin-5-one
1-(piperid-1-yl)-7-bromo-4-(3-phenylallyl)-4H-[1,2,4]triazolo[4,3-a]quinazolin-5-one
4-(7-Bromo-1-dimethylamino-5-oxo-5H-[1,2,4]triazolo[4,3-a]qu inazolin-4-ylmethyl)benzonitrile
4-(Bromo-dimethylaminooxo-5H-[1,2,4]triazolo[4,3-a]quinazolin-4-ylmethyl)benzoic acid methyl ester
7-Bromo-1-dimethylamino-4-(3-phenylallyl)-4H-[1,2,4]triazolo[4,3-a]quinazolin-5-one
7-Bromo-1-dimethylamino-4-(3-phenylprop-2-ynyl)-4H-[1,2,4]triazolo[4,3-a]quinazolin-5-one
1-Azepan-1-yl-7-methyl-4-(3-phenylallyl)-4H-[1,2,4]triazolo[4,3-a]quinazolin-5-one
4-(3,4-Dimethoxybenzyl)-7-methyl-1-pyrrolidin-1-yl-4H-[1,2,4]triazolo[4,3-a]quinazolin-5-one
7-Methyl-4-(3-phenylallyl)-1-pyrrolidin-1-yl-4H-[1,2,4]triazolo[4,3-a]quinazolin-5-one
7-Amino-4-((E)-3-phenylallyl)-1-pyrrolidin-1-yl-4H-[1,2,4]triazolo[4,3-a]quinazolin-5-one
7-Amino-4-((E)-3-pyrid-3-ylallyl)-1-pyrrolidin-1-yl-4H-[1,2,4]triazolo[4,3-a]quinazolin-5-one
4-Benzyl-7-methylamino-1-pyrrolidin-1-yl-4H-[1,2,4]triazolo[4,3-a]quinazolin-5-one
4-(7-Methylamino-5-oxo-1-pyrrolidin-1-yl-5H-[1,2,4]triazolo[4,3-a]quinazolin-4-ylmethyl)benzonitrile
[4-(7-Bromo-5-oxo-1-pyrrolidin-1-yl-5H-[1,2,4]triazolo[4,3-a]quinazolin-4-ylmethyl)phenyl]acetic acid methyl ester
2-[4-(7-Bromo-5-oxo-1-pyrrolidin-1-yl-5H-[1,2,4]triazolo[4,3-a]quinazolin-4-ylmethyl)phenyl]-N-methyacetamide
2-[4-(7-Bromo-5-oxo-1-pyrrolidin-1-yl-5H-[1,2,4]triazolo[4,3-a]quinazolin-4-ylmethyl)phenyl]-N,N-dimethylacetamide
1-Dimethylamino-7-methyl-4-(3-pyrid-3-ylallyl)-4H-[1,2,4]triazolo[4,3-a]quinazoline-5-thione

Other preferred compounds are those disclosed in the PCT Application WO 98/49169 and most preferably the diazepino-indolones are preferred and more particularly, the following compounds :
(3S)3-(2-methyl-4oxo-4H-quinazolin-3yl)-1-phenyl-6,7-dihydro-3H[1,4]diazepino[6,7,1-*hi*]indol-4-one.
9-methoxy-3-(2-methyl-4-oxo-4H-quinazolin-3yl)-1-phenyl-6,7-dihydro-3H-[1,4]diazepino[6,7,1-*hi*]indol-4-one.
2-methyl-3-(9-methoxy-4-oxo-1-phenyl-3,4,6,7-tetrahydro-[1,4]diazepino[6,7,1-*hi*]indol-3-yl)-4-oxo-3,4-dihydro-quinazoline-7-carboxylic methyl ester.
2-methyl-3-(9-methoxy-4-oxo-1-phenyl-3,4,6,7-tetrahydro-[1,4]diazepino [6,7,1-*hi*]indol-3-yl)-4-oxo-3,4-dihydro-quinazoline-7-carboxylic acid.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylic acid.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylic acid.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylic acid.
D-glucamine 2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylate salt.
(3R) 2-methyl-3-(9-methyl-4-oxo-1-phenyl-3,4,6,7-tetrahydro-[1,4]diazepino [6,7,1-*hi*]indol-3-yl)-4-oxo-3,4-dihydro-quinazoline-7-carboxylic acid.
(3S) 2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7- carboxylic acid.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylique acide 2-morpholin-4-yl-ethyl ester.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylate salt of 2-acetoxyethyle.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylate salt of 2-methoxyethyle.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylate salt of 2-hydroxyethyle.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylate salt of 1-(2S)-glycerol.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylate salt of 2-hydroxy3-morpholin-4-yl-propyle.
9-methyl-3-(2-methyl-4-oxo-4H-quinazolin-3yl)-1-phenyl-6,7-dihydro-3H-[1,4] diazepino[6,7,1-*hi*]indol-4-one.
(3S)9-methyl-3-(2,5-dimethyl-4-oxo-4H-quinazolin-3yl)-1-phenyl-6,7-dihydro-3H-[1,4]diazepino[6,7,1-*hi*]indol-4-one.
(3S)9-methyl-3-(2,6-dimethyl-4-oxo-4H-quinazolin-3yl)-1-phenyl-6,7-dihydro-3H-[1,4]diazepino[6,7,1-*hi*]indol-4-one.
(3S)9-methyl-3-(2,6-dimethyl-4-oxo-4H-quinazolin-3yl)-1-phenyl-6,7-dihydro-3H-[1,4]diazepino[6,7,1-*hi*]indol-4-one.
(3S)9-methyl-3-(2,8-dimethyl-4-oxo-4H-quinazolin-3yl)-1-phenyl-6,7-dihydro-3H-[1,4]diazepino[6,7,1-*hi*]indol-4-one.
3-(5-methoxy-2-methyl-4-oxo-4H-quinazolin-3yl)-9-methyl-1-phenyl-6,7-dihydro-3H-[1,4]diazepino[6,7,1-*hi*]indol-4-one.
3-(7-methoxy-2-methyl-4-oxo-4H-quinazolin-3yl)-9-methyl-1-phenyl-6,7-dihydro-3H-[1,4]diazepino[6,7,1-*hi*]indol-4-one.
3-(6-methoxy-2-methyl-4-oxo-4H-quinazolin-3yl)-9-methyl-1-phenyl-6,7-dihydro-3H-[1,4]diazepino[6,7,1-*hi*]indol-4-one.
3-(6-bromo-2-methyl-4-oxo-4H-quinazolin-3yl)-9-methyl-1-phenyl-6,7-dihydro-3H-[1,4]diazepino[6,7,1-*hi*]indol-4-one.
3-(5-hydroxymethyl-2-methyl-4-oxo-4H-quinazolin-3yl)-9-methyl-1-phenyl-6,7-dihydro-3H-[1,4]diazepino[6,7,1-*hi*]indol-4-one.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-5-carboxylic acid tertiobutylic ester.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-5-carboxylic acid.
3-(7-hydroxymethyl-2-methyl-4-oxo-4H-quinazolin-3-yl)-9-methyl-1-phenyl-6,7-dihydro-3H-[1,4]diazepino[6,7,1-*hi*]indol-4-one.
(3S)3-(5-fluoro-2-methyl-4-oxo-4H-quinazolin-3-yl)-9-methyl-1-phenyl-6,7-dihydro-3H-[1,4]diazepino[6,7,1-*hi*]indol-4-one.
3-(5-chloro-2-methyl-4-oxo-4H-quinazolin-3-yl)-9-methyl-1-phenyl-6,7-dihydro-3H-[1,4]diazepino[6,7,1-*hi*]indol-4-one.
(9-methyl-3-oxo-4*H*-quinazolin-3-yl)-1-phenyl-6,7-dihydro-3H-[1,4]diazepino [6,7,1-*hi*]indol-4-one.
3-(9-methyl-4-oxo-1-phenyl-3,4,6,7-tetrahydro-[1,4]diazepino[6,7,1-*hi*]indol-3-yl)-4-oxo-3,4-dihydro-quinazoline-2-carboxylic acid ethyl ester.
5-chloro-3-(9-methyl-4-oxo-1-phenyl-3,4,6,7-tetrahydro-[1,4] diazepino[6,7,1-*hi*]indol-3-yl)-4-oxo-3,4-dihydro-quinazolin-2- carboxylic acid ethyl ester.
[3-(9-methyl-4-oxo-1-phenyl-3,4,6,7-tetrahydro-[1,4]diazepino[6,7,1-*hi*]indol-3-yl)-4-oxo-3,4-dihydro-quinazolin-2-yl]- acetic acid ethyl ester.
Acetic acid 3-(9-methyl-4-oxo-1-phenyl-3,4,6,7-tetrahydro-[1,4]diazepino[6,7,1-*hi*]indol-3-yl)-4-oxo-3,4-dihydro-qu inazolin-2-ylmethyl ester.
9-methyl-3-(2-methyloxymethyl-4-oxo-4H-quinazolin-3yl)-1-phenyl-6,7-dihydro-3H-[1,4]diazepino[6,7,1-*hi*]indol-4-one.
9-methyl-3-(2-hydroxymethyl-4-oxo-4H-quinazolin-3yl)-1-phenyl-6,7-dihydro-3H-[1,4]diazepino[6,7,1-*hi*]indol-4-one.
Succinic acid mono of [[2-(2-methoxy-ethoxy)-ethoxy] and [3-(9-methyl-4-oxo-1-phenyl-3,4,6,7-tetrahydro-[1,4]diazepino[6,7,1-*hi*]indol-3-yl)-4-oxo-3,4-dihydro-quinazolin-2-yl]-methyl] ester
Acetic acid mono of [2-(2-methoxy-ethoxy)-ethoxy] and [3-(9-methyl-4-oxo-1-phenyl-3,4,6,7-tetrahydro-[1,4]diazepino[6,7,1-*hi*]indol-3-yl)-4-oxo-34-dihydroquinazolin-2-yl]- methyl] ester.
N-(2-morpholin-4-yl-ethyl)-succinamic acid 3-(9-methyl-4-oxo-1-phenyl-3,4,6,7-tetrahydro-[1,4]diazepino[6,7,1-*hi*]indol-3-yl)-4-oxo-3,4-dihydroquinazolin-2-yl-methyl ester.
Succinic acid 3-(9-methyl-4-oxo-1-phenyl-3,4,6,7-tetrahydro-[1,4] diazepino[6,7,1-*hi*]indol-3-yl)-4-oxo-3,4-dihydro-quinazolin-2-ylmethyl ester 2-morpholin-4-yl-ethyl ester.
6-*t*-butoxycarbonylamino-2-{3-[3-(9-methyl-4-oxo-1-phenyl-3,4,6,7-tetrahydro-[1,4]diazepino[6,7,1-*hi*]indol-3-yl)-4-oxo-3,4-dihydro-quinazolin-2-yl methoxycarbonyl]-propionylamino}-hexanoïc acid methyl ester.
3-[3-(9-methyl-4-oxo-1-phenyl-3,4,6,7-tetrahydro-[1,4]diazepino[6,7,1-*hi*]indol-3-yl)-4-oxo-3,4-dihydro-quinazolin-2-yl]-propanoic acid ethyl ester.
(3S) 3-(2-methyl-4-oxo-4*H*-pyrido[3,4-d]pyrimidin-3-yl)-9-methyl-1-phenyl-6,7-dihydro-3*H*-[1,4]diazepino[6,7,1-*hi*]indol-1-one.
3-(2-methyl-4-oxo-4*H*-pteridin-3-yl)-9-methyl-1-phenyl-6,7-dihydro-3*H*-[1,4] diazepino[6,7,1-*hi*]indol-4-one.
9-amino-3-(2-methyl-4-oxo-4H-pyrido[3,4-d]pyrimidin-3-yl)-1-phenyl-6,7-dihydro-3H-[1,4]diazepino[6,7,1-*hi*]indol-4-one.
3-(2-methyl-4-oxo-4H-quinazolin-3-yl)-9-nitro-1-phenyl-6,7-dihydro-3H-[1,4]diazepino[6,7,1-*hi*]indol-4-one.
3-(2,5-dimethyl-4-oxo-4H-quinazolin-3-yl)-9-nitro-1-phenyl-6,7-dihydro-3H-[1,4]diazepino[6,7,1-*hi*]indol-4-one.
3-(2,6-dimethyl-4-oxo-4H-quinazolin-3-yl)-9-nitro-1-phenyl-6,7-dihydro-3H-[1,4]diazepino[6,7,1-*hi*]indol-4-one.
3-(2,7-dimethyl-4-oxo-4H-quinazolin-3-yl)-9-nitro-1-phenyl-6,7-dihydro-3H-[1,4]diazepino[6,7,1-*hi*]indol-4-one.
3-(2,8-dimethyl-4-oxo-4H-quinazolin-3-yl)-9-nitro-1-phenyl-6,7-dihydro-3H-[1,4]diazepino[6,7,1-*hi*]indol-4-one.
3-(2,6,8-trimethyl-4-oxo-4H-quinazolin-3-yl)-9-nitro-1-phenyl-6,7-dihydro-3H-[1,4]diazepino[6,7,1-*hi*]indol-4-one.
3-(8-bromo-2,6-dimethyl-4-oxo-4H-quinazolin-3-yl)-9-nitro-1-phenyl-6,7-dihydro-3H-[1,4]diazepino[6,7,1-*hi*]indol-4-one.
(3S)3-(2-methyl-4-oxo-4H-pyrido[3,4-*d*]pyrimidin-3-yl)-9-nitro-1-phenyl-6,7-dihydro-3H-[1,4]diazepino[6,7,1-*hi*]indol-4-one.
5-chloro-2-methyl-3-(9-nitro-4-oxo-1-phenyl-3,4,6,7-tetrahydro-[1,4]diazepino[6,7,1-*hi*]indol-3-yl)-4-oxo-3,4-dihydro-quinazoline.
(3S)9-amino-3-(2-methyl-4-oxo-4H-quinazolin-3-yl)-1-phenyl-6,7-dihydro-3H-[1,4]diazepino[6,7,1-*hi*]indol-4-one.
3-(9-amino-4-oxo-1-phenyl-3,4,6,7-tetrahydro-[1,4]diazepino[6,7,1-*hi*]indol-3-yl)-2-methyl-4-oxo-3,4-dihydro-quinazoline-7-carboxylic acid methyl ester and their enantiomers.
3-(9-amino-4-oxo-1-phenyl-3,4,6,7-tetrahydro-[1,4]diazepino[6,7,1-*hi*]indol-3-yl)-2-methyl-4-oxo-3,4-dihydro-quinazoline-7-carboxylic acid.
9-amino-3-(2-methyl-4-oxo-4H-quinazolin-3-yl)-1-phenyl-6,7-dihydro-3H-[1,4] diazepino[6,7,1-*hi*]indol-4-one.
9-amino-3-(2,5-dimethyl-4-oxo-4H-quinazolin-3-yl)-1-phenyl-6,7-dihydro-3H-[1,4]diazepino[6,7,1-*hi*]indol-4-one.
9-amino-3-(2,6-dimethyl-4-oxo-4H-quinazolin-3-yl)-1-phenyl-6,7-dihydro-3H-[1,4]diazepino[6,7,1-*hi*]indol-4-one.
9-amino-3-(2,8-dimethyl-4-oxo-4H-quinazolin-3-yl)-1-phenyl-6,7-dihydro-3H-[1,4]diazepino[6,7,1-*hi*]indol-4-one.
9-amino-3-(2,6,8-trimethyl-4-oxo-4H-quinazolin-3-yl)-1-phenyl-6,7-dihydro-3H-[1,4]diazepino[6,7,1-*hi*]indol-4-one.
9-amino-3-(5-chloro-2-methyl-4-oxo-4*H*-quinazolin-3-yl)-1-phenyl-6,7-dihydro-3*H*-[1,4]diazepino[6,7,1-*hi*]indol-4-one.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxamide.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carbonitrile.
9-methyl-3-[2-methyl-4-oxo-7-(1*H*-tetrazol-5-yl)-4*H*-quinazolin-3-yl]-1-phenyl-6,7-dihydro-3*H*-[1,4]diazepino[6,7,1-*hi*]indol-4-one.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-N-methylcarboxamide.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-N-dimethylcarboxamide.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylic acid (2-morpholin-4-yl-ethyl)-amide.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylic acid (3-(morpholin-4yl-propyl)-amide.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-(2-amino-)-ethylcarboxamide and its hydrochloride salt.
3-{7-[4-(2-hydroxy-ethyl)-piperazine-1-carbonyl]-2-methyl-4-oxo-4*H*-quinazolin-3-yl}-9-methyl-1-phenyl-6,7-dihydro-3*H*-[1,4]diazepino[6,7,1-*hi*]indol-4-one and its hydrochloride salt.
(2S) 6-*t*-butoxycarbonylamino-2-{[2-methyl-3-(9-methyl-4-oxo-1-phenyl-3,4,6,7-tetrahydro-[1,4]diazepino[6,7,1-*hi*]indol-3-yl)-4-oxo-3,4-dihydroquinazolin-7-carbonyl]-amino}-hexanoïc acid methyl ester.
(2S) 6-amino-2-{[2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazolin-7-carbonyl]amino}-hexanoïc acid methyl ester.
2-methyl-3-(9-methyl-4-oxo-1-phenyl-3,4,6,7-tetrahydro-[1,4]diazepino[6,7,1-*hi*]indol-3-yl)-4-oxo-3,4-dihydro-quinazoline-7-carboxylic acid pentafluorophenyl ester.
N-( 2-methyl-3-(9-methyl-4-oxo-1-phenyl-3,4,6,7-tetrahydro-[1,4]diazepino [6,7,1-*hi*]indol-3-yl)-4-oxo-3,4-dihydro-quinazoline-7-carboxyl-)-tyrosine.
N-( 2-methyl-3-(9-methyl-4-oxo-1-phenyl-3,4,6,7-tetrahydro-[1,4]diazepino [6,7,1-*hi*]indol-3-yl)-4-oxo-3,4-dihydro-quinazoline-7-carboxyl-)-alanine.
N-( 2-methyl-3-(9-methyl-4-oxo-1-phenyl-3,4,6,7-tetrahydro-[1,4]diazepino [6,7,1-*hi*]indol-3-yl)-4-oxo-3,4-dihydro-quinazoline-7-carboxyl-)-phenyl-alanine.
N-( 2-methyl-3-(9-methyl-4-oxo-1-phenyl-3,4,6,7-tetrahydro-[1,4]diazepino [6,7,1-*hi*]indol-3-yl)-4-oxo-3,4-dihydro-quinazoline-7-carboxyl-)-glycine.
N-( 2-methyl-3-(9-methyl-4-oxo-1-phenyl-3,4,6,7-tetrahydro-[1,4]diazepino [6,7,1-*hi*]indol-3-yl)-4-oxo-3,4-dihydro-quinazoline-7-carboxyl-)-leucine.
N-( 2-methyl-3-(9-methyl-4-oxo-1-phenyl-3,4,6,7-tetrahydro-[1,4]diazepino [6,7,1-*hi*]indol-3-yl)-4-oxo-3,4-dihydro-quinazoline-7-carboxyl-)-aspartic acid.
N-( 2-methyl-3-(9-methyl-4-oxo-1-phenyl-3,4,6,7-tetrahydro-[1,4]diazepino [6,7,1-*hi*]indol-3-yl)-4-oxo-3,4-dihydro-quinazoline-7-carboxyl-)-glutamic acid.
N-( 2-methyl-3-(9-methyl-4-oxo-1-phenyl-3,4,6,7-tetrahydro- [1,4]diazepino [6,7,1-*hi*]indol-3-yl)-4-oxo-3,4-dihydro-quinazoline-7-carboxyl-)-lysine.
2-[2-methyl-3-(9-methyl-4-oxo-1-phenyl-3,4,6,7-tetrahydro-[1,4]diazepino [6,7,1-*hi*]indol-3-yl)-4-oxo-3,4-dihydro-quinazoline-7-carbonyl]-amino}-3-phenyl-(L)-propionique acide ethyl ester.
N-( 2-methyl-3-(9-methyl-4-oxo-1-phenyl-3,4,6,7-tetrahydro-[1,4]diazepino [6,7,1-*hi*]indol-3-yl)-4-oxo-3,4-dihydro-quinazoline-7-carboxyl-)-proline.

Other preferred compounds are those disclosed in the PCT Application N° PCT/EP00/13380 as substituted pyrazolo-[4,3-*e*]diazepines and most preferably the following compounds :
1-Ethyl-5-(4-methoxy-phenyl)-3-methyl-6,7-dihydro-1*H*-pyrazolo[4,3-*e*][1,4]diazepin-8-one,
5-(4-Bromo-phenyl)-1-ethyl-3-methyl-6,7-dihydro-1*H*-pyrazolo[4,3-*e*][1,4]diazepin-8-one,
1,3-Dimethyl-5-phenyl-6,7-dihydro-1*H*-pyrazolo[4,3-e][1,4]diazepin-8-one,
5-(4-Methoxy-phenyl)-1,3-dimethyl-6,7-dihydro-1*H*-pyrazolo[4,3-*e*][1,4]diazepin-8-one,
5-(4-Bromo-phenyl)-1,3-dimethyl-6,7-dihydro-1*H*-pyrazolo[4,3-*e*][1,4]diazepin-8-one,
1-Ethyl-3-methyl-5-naphthalen-2-yl-6,7-dihydro-1*H*-pyrazolo[4,3-*e*][1 ,4]diazepin-8-one,
5-(3-Chloro-thiophen-2-yl)-1-ethyl-3-methyl-6,7-dihydro-1*H*-pyrazolo[4,3-*e*][1,4]diazepin-8-one,
3-(1-Ethyl-3-methyl-8-oxo-1,6,7,8-tetrahydro-pyrazolo[4,3-e][1,4]diazepin-5yl)-propanoate de methyle,
1-Ethyl-5-phenyl-6,7-dihydro-1*H*-pyrazolo[4,3-*e*][1,4]diazepin-8-one,
5-(4-Chloro-phenyl)-1-ethyl-3-methyl-6,7-dihydro-1*H*-pyrazolo[4,3-*e*][1,4]diazepin-8-one,
5-(4-Amino-phenyl)-1-ethyl-3-methyl-6,7-dihydro-1*H*-pyrazolo[4,3-e][1,4]diazepin-8-one,
1-Ethyl-5-(4-fluoro-phenyl)-3-methyl-6,7-dihydro-1*H*-pyrazolo[4,3-e][1,4]diazepin-8-one,
5-(3-Bromo-phenyl)-1-ethyl-3-methyl-6,7-dihydro-1*H*-pyrazolo[4,3-e][1,4]diazepin-8-one,
3-Methyl-5-phenyl-1-propyl-6,7-dihydro-1*H*-pyrazolo[4,3-*e*][1,4]diazepin-8-one,
1-Isopropyl-3-methyl-5-phenyl-6,7-dihydro-1 H-pyrazolo[4,3-*e*][1,4]diazepin-8-one,
(±)1-Ethyl-3-methyl-5-phenyl-4,5,6,7-tetrahydro-1*H*-pyrazolo[4,3-*e*][1,4]diazepin-8-one,
1-(2-Hydoxy-ethyl)-3-methyl-5-phenyl-6,7-dihydro-1*H*-pyrazolo[4,3-*e*][1 ,4]diazepin-8-one,
1-Ethyl-3-methyl-5-phenyl-6,7-dihydro-1*H*-pyrazolo[4,3-*e*][1,4]diazepin-8-thione,
1-Ethyl-5-(4-methoxy-phenyl)-6,7-dihydro-1*H*-pyrazolo[4,3-*e*][1,4]diazepin-8-one,
1-Ethyl-5-(3-methoxy-phenyl)-3-methyl-6,7-dihydro-1*H*-pyrazolo[4,3-*e*][1,4]diazepin-8-one,
5-(2-Amino-phenyl)-1-ethyl-3-methyl-6,7-dihydro-1*H*-pyrazolo[4,3-*e*][1,4]diazepin-8-one,
1-Ethyl-5-(2-methoxy-phenyl)-3-methyl-6,7-dihydro-1*H*-pyrazolo[4,3-*e*][1,4]diazepin-8-one,
1-Ethyl-3-isopropyl-5-phenyl-6,7-dihydro-1 *H*-pyrazolo[4,3*-e*][1,4]diazepin-8-one,
3-*tert*-Butyl-1-ethyl-5-phenyl-6,7-dihydro-1 *H*-pyrazolo[4,3-*e*][1 ,4]diazepin-8-one,
1-Ethyl-3-methyl-5-phenyl-4,5,6,7-tetrahydro-1*H*-pyrazolo[4,3-*e*][1,4]diazepin-8-one isomère 1,
1-Ethyl-3-methyl-5-phenyl-4,5,6,7-tetrahydro-1*H*-pyrazolo[4,3-*e*][1,4]diazepin-8-one isomère 2,
3-Methyl-5-phenyl-1-propyl-6,7-dihydro-1*H*-pyrazolo[4,3-*e*][1,4]diazepin-8-thione,
1 -Ethyl-3-methyl-5-p-tolyl-6,7-dihydro-1*H*-pyrazolo[4,3-e][1,4]diazepin-8-one, 5-(3-Amino-phenyl)-1-ethyl-3-methyl-6,7-dihydro-1*H*-pyrazolo[4,3-*e*][1,4]diazepin-8-one,
3-*tert*-Butyl-1-ethyl-5-phenyl-6,7-dihydro-1H-pyrazolo[4,3-e][1,4]diazepin-8-thione,
1 -Ethyl-3-isopropyl-5-phenyl-6,7-dihydro-1 *H*-pyrazolo[4,3-*e*][1,4]diazepin-8-thione,
5-(4-Amino-phenyl)-3-*tert*-butyl-1-ethyl-6,7-dihydro-1*H*-pyrazolo[4,3-*e*][1,4]diazepin-8-one,
5-(4-Amino-phenyl)-1-ethyl-3-isopropyl-6,7-dihydro-1*H*-pyrazolo[4,3-*e*][1,4]diazepin-8-one,
1-Ethyl-5-(4-hydroxy-phenyl)-3-methyl-6,7-dihydro-1*H*-pyrazolo[4,3-*e*][1,4]diazepin-8-one,
5-(4-Amino-phenyl)-3-methyl-1-propyl-6,7-dihydro-1*H*-pyrazolo[4,3-*e*][1,4]diazepin-8-one,
3-Methyl-5-phenyl-1-(2,2,2-trifluoro-ethyl)-6,7-dihydro-1*H*-pyrazolo[4,3-*e*][1,4]diazepin-8-one,
1,5-Diethyl-3-methyl-6,7-dihydro-1*H*-pyrazolo[4,3-*e*][1,4]diazepin-8-one,
5-Cyclohexyl-1-ethyl-3-methyl-6,7-dihydro-1*H*-pyrazolo[4,3-*e*][1,4]diazepin-8-one,
1-Ethyl-3-methyl-5-pyridin-4-yl-6,7-dihydro-1 *H*-pyrazolo[4,3-*e*][1,4]diazepin-8-one,
(±)1-Ethyl-3-methyl-5-pyridin-4-yl-4,5,6,7-tetrahydro-1*H*-pyrazolo[4,3-*e*][1 ,4]diazepin-8-one,
5-*tert*-Butyl-1-ethyl-3-methyl-4,5,6,7-tetrahydro-1*H*-pyrazolo[4,3-*e*][1,4]diazepin-8-one,
5-(4-Diethylamino-phenyl)-1-ethyl-3-methyl-6,7-dihydro-1*H*-pyrazolo[4,3-*e*][1,4]diazepin-8-one,
3-Butyl-1-ethyl-5-phenyl-6,7-dihydro-1*H*-pyrazolo[4,3-*e*][1,4]diazepin-8-one, *N*-[4-(1-Ethyl-3-methyl-8-oxo-1,6,7,8-tetrahydro-pyrazolo[4,3-*e*][1,4]diazepin-5-yl)-phenyl]-acetamide,
4-(1-Ethyl-3-methyl-8-oxo-1,6,7,8-tetrahydro-pyrazolo[4,3*-e*][1,4]diazepin-5-yl)-benzonitrile,
5-*tert*-Butyl-1-ethyl-3-isopropyl-6,7-dihydro-1*H*-pyrazolo[4,3-*e*][1,4]diazepin-8-one,
1-Ethyl-3-isopropyl-5-p-tolyl-6,7-dihydro-1*H*-pyrazolo[4,3-*e*][1,4]diazepin-8-one,
4-(1-Ethyl-3-isopropyl-8-oxo-1,6,7,8-tetrahydro-pyrazolo[4,3-*e*][1,4]diazepin-5-yl)-benzonitrile,
1-Ethyl-3-isopropyl-5-(4-pyrrolidin-1-yl-phenyl)-6,7-dihydro-1*H*-pyrazolo[4,3-*e*][1,4]diazepin-8-one,
5-(2,4-Dimethoxy-phenyl)-1-ethyl-3-isopropyl-6,7-dihydro-1*H*-pyrazolo[4,3-*e*][1,4]diazepin-8-one,
5-*tert*-Butyl-3-isopropyl-1-propyl-6,7-dihydro-1*H*-pyrazolo[4,3-*e*][1,4]diazepin-8-one,
3-Isopropyl-1-propyl-5-p-tolyl-6,7-dihydro-1*H*-pyrazolo[4,3-*e*][1,4]diazepin-8-one,
4-(3-Isopropyl-8-oxo-1-propyl-1,6,7,8-tetrahydro-pyrazolo[4,3-*e*][1,4]diazepin-5-yl)-benzonitrile,
3-lsopropyl-1-propyl-5-(4-pyrrolidin-1-yl-phenyl)-6,7-dihydro-1*H*-pyrazolo[4,3-*e*][1,4]diazepin-8-one,
5-(2,4-Dimethoxy-phenyl)-3-isopropyl-1-propyl-6,7-dihydro-1*H*-pyrazolo[4,3-*e*][1,4]diazepin-8-one,
3,5-Di-*tert*-butyl-1-ethyl-6,7-dihydro-1*H*-pyrazolo[4,3-*e*][1,4]diazepin-8-one, 3-*tert*-Butyl-1-ethyl-5-p-tolyl-6,7-dihydro-1*H*-pyrazolo[4,3*-e*][1,4]diazepin-8-one,
4-(3-*tert*-Butyl-1-ethyl-8-oxo-1,6,7,8-tetrahydro-pyrazolo[4,3*-e*][1,4]diazepin-5-yl)-benzonitrile,
3-*tert*-Butyl-1-ethyl-5-(4-pyrrolidin-1-yl-phenyl)-6,7-dihydro-1*H*-pyrazolo[4,3-*e*][1,4]diazepin-8-one,
3-*tert*-Butyl-5-(2,4-dimethoxy-phenyl)-1-ethyl-6,7-dihydro-1*H*-pyrazolo[4,3-*e*][1,4]diazepin-8-one,
3,5-Di-*tert*-butyl-1-propyl-6,7-dihydro-1 *H*-pyrazolo[4,3*-e*][1,4]diazepin-8-one, 3-*tert*-Butyl-1-propyl-5-p-tolyl-6,7-dihydro-1*H*-pyrazolo[4,3*-e*][1,4]diazepin-8-one,
4-(3-*tert*-Butyl-8-oxo-1-propyl-1,6,7,8-tetrahydro-pyrazolo[4,3*-e*][1,4]diazepin-5-yl)-benzonitrile,
3-tert-Butyl-1-propyl-5-(4-pyrrolidin-1-yl-phenyl)-6,7-dihydro-1*H*-pyrazolo[4,3-*e*][1,4]diazepin-8-one,
3-*tert*-Butyl-5-(2,4-dimethoxy-phenyl)-1-propyl-6,7-dihydro-1*H*-pyrazolo[4,3-*e*][1,4]diazepin-8-one,
3-Butyl-1-ethyl-5-phenyl-6,7-dihydro-1*H*-pyrazolo[4,3-*e*][1,4]diazepin-8-thione,
1-Ethyl-3-methyl-5-pyridin-3-yl-6,7-dihydro-1*H*-pyrazolo[4,3-*e*][1,4]diazepin-8-one,
1-Ethyl-3-methyl-5-pyridin-2-yl-6,7-dihydro-1*H*-pyrazolo[4,3-*e*][1,4]diazepin-8-one,
1-Ethyl-3-methyl-5-phenyl-1,6-dihydro-pyrazolo[4,3-*e*][1,4]diazepin-8-yl-cyanamide,
*N*-[4-(1-Ethyl-3-methyl-8-oxo-1,6,7,8-tetrahydro-pyrazolo[4,3-*e*][1,4]diazepin-5-yl)-phenyl]-(phenylsulfonyl)benzenesulfonamide,
(1-Ethyl-3-methyl-5-phenyl-1,6-dihydro-pyrazolo[4,3-e][1,4]diazepin-8-yl)-methyl-amine,
1-Ethyl-3-isopropyl-5-phenyl-6,7-dihydro-1*H*-pyrazolo[4,3-*e*][1,4]diazepin-8-ylidène-cyanamide,
3-*tert*-Butyl-1-ethyl-5-phenyl-6,7-dihydro-1*H*-pyrazolo[4,3-e][1,4]diazepin-8-ylidène-cyanamide,
1-Cyclopentyl-3-methyl-5-phenyl-6,7-dihydro-1*H*-pyrazolo[4,3-*e*][1 ,4]diazepin-8-one,
1-Cyclopropylmethyl-3-methyl-5-phenyl-6,7-dihydro-1*H*-pyrazolo[4,3-*e*][1,4]diazepin-8-one,
1-Cyclobutylmethyl-3-methyl-5-phenyl-6,7-dihydro-1*H*-pyrazolo[4,3-*e*][1,4]diazepin-8-one
1 -Allyl-3-methyl-5-phenyl-6,7-dihydro-1*H*-pyrazolo[4,3*-e*][1,4]diazepin-8-one,
1-Ethyl-3-methyl-5-(4-trifluoromethyl-phenyl)-6,7-dihydro-1*H*-pyrazolo[4,3-*e*][1,4]diazepin-8-one,
1-Ethyl-3-isopropyl-5-(4-trifluoromethyl-phenyl)-6,7-dihydro-1*H*-pyrazolo[4,3-*e*][1,4]diazepin-8-one,
3-*tert-*Butyl-1-ethyl-5-(4-trifluoromethyl-phenyl)-6,7-dihydro-1*H*-pyrazolo[4,3-*e*][1 ,4]diazepin-8-one,
3-Isopropyl-1-propyl-5-*p*-tolyl-6,7-dihydro-1*H*-pyrazolo[4,3-*e*][1,4]diazepin-8-thione,
3,5-Di-*tert*-butyl-1-propyl-6,7-dihydro-1*H*-pyrazolo[4,3-*e*][1,4]diazepin-8-thione,
5*-tert*-Butyl-3-isopropyl-1-propyl-6,7-dihydro-1*H*-pyrazolo[4,3-*e*][1,4]diazepin-8-thione,
1 -Ethyl-3-isopropyl-5-p-tolyl-6,7-dihydro-1*H*-pyrazolo[4,3-e][1,4]diazepin-8-thione,
5-*tert*-Butyl-1-ethyl-3-isopropyl-6,7-dihydro-1*H*-pyrazolo[4,3-e][1,4]diazepin-8-thione,
(±)3-sec-Butyl-1-ethyl-5-phenyl-6,7-dihydro-1*H*-pyrazolo[4,3-*e*][1,4]diazepin-8-one,
(±)3-sec-Butyl-1-ethyl-5-pyridin-4-yl-6,7-dihydro-1*H*-pyrazolo[4,3-e][1 ,4]diazepin-8-one,
(±)3-sec-Butyl-5-phenyl-1-propyl-4-yl-6,7-dihydro-1*H*-pyrazolo[4,3-*e*][1,4]diazepin-8-one,
(±)3-sec-Butyl-1-propyl-5-pyridin-4-yl-6,7-dihydro-1*H*-pyrazolo[4,3-*e*][1,4]diazepin-8-one,
3-Cyclohexyl-1-ethyl-5-phenyl-4-yl-6,7-dihydro-1*H*-pyrazolo[4,3-*e*][1,4]diazepin-8-one,
3-Cyclohexyl-1-ethyl-5-pyridin-4-yl-6,7-dihydro-1*H*-pyrazolo[4,3-*e*][1,4]diazepin-8-one,
3-Cyclohexyl-5-phenyl-1-propyl-4-yl-6,7-dihydro-1*H*-pyrazolo[4,3-*e*][1,4]diazepin-8-one,
3-Cyclohexyl-1-propyl-5-pyridin-4-yl-6,7-dihydro-1 *H*-pyrazolo[4,3-*e*][1 ,4]diazepin-8-one,
3-Cyclohexylmethyl-1-ethyl-5-pyridin-4-yl-6,7-dihydro-1*H*-pyrazolo[4,3-*e*][1,4]diazepin-8-one,
3-Cyclohexylmethyl-5-phenyl-1-propyl-6,7-dihydro-1*H*-pyrazolo[4,3-*e*][1,4]diazepin-8-one,
3-Cyclohexylmethyl-1-propyl-5-pyridin-4-yl-6,7-dihydro-1*H*-pyrazolo[4,3-*e*][1,4]diazepin-8-one,
3-Cyclohexylmethyl-1-ethyl-5-phenyl-6,7-dihydro-1*H*-pyrazolo[4,3-*e*][1 ,4]diazepin-8-one,
1-Ethyl-8-oxo-5-phenyl-1,6,7,8-tetrahydro-pyrazolo[4,3*-e*][1,4]diazepine-3-carboxylic acid ethyl ester,
5-*tert*-Butyl-1-ethyl-3-isopropyl-6,7-dihydro-1*H*-pyrazolo[4,3*-e*][1,4]diazepin-8-ylidene-cyanamide,
1-Ethyl-3-isopropyl-5-p-tolyl-6,7-dihydro-1*H*-pyrazolo[4,3*-e*][1,4]diazepin-8-ylidene-cyanamide,
1-Ethyl-3-isopropyl-5-(4-methoxy-phenyl)-6,7-dihydro-1*H*-pyrazolo[4,3-*e*][1,4]diazepin-8-one,
1-Ethyl-5-(4-methoxy-phenyl)-3-methyl-6,7-dihydro-1*H*-pyrazolo[4,3-*e*][1,4]diazepine-8-thione,
1-Ethyl-5-(4-methoxy-phenyl)-3-methyl-6,7-dihydro-1*H*-pyrazolo[4,3-*e*][1,4]diazepin-8-ylidene-cyanamide,
3-Isopropyl-5-(4-methoxy-phenyl)-1-propyl-6,7-dihydro-1*H*-pyrazolo[4,3*e*][1,4]diazepin-8-one,
3-*tert*-Butyl-1-ethyl-5-(4-methoxy-phenyl)-6,7-dihydro-1*H*-pyrazolo[4,3-*e*][1,4]diazepin-8-one,
3-*tert*-Butyl-5-(4-methoxy-phenyl)-1-propyl-6,7-dihydro-1*H*-pyrazolo[4,3-*e*][1,4]diazepin-8-one,
1-Ethyl-5-(4-hydroxy-phenyl)-3-methyl-6,7-dihydro-1*H*-pyrazolo[4,3-*e*][1,4]diazepin-8-ylidene-cyanamide,
(±)3-sec-Butyl-1-ethyl-5-(4-methoxy-phenyl)-6,7-dihydro-1*H*-pyrazolo[4,3-*e*][1,4]diazepin-8-one,
(±)3sec-Butyl-5-(4-methoxy-phenyl)-1-propyl-6,7-dihydro-1*H*-pyrazolo[4,3-*e*][1,4]diazepin-8-one,
1-Ethyl-3-methyl-5-(3,4,5-trimethoxy-phenyl)-6,7-dihydro-1*H*-pyrazolo[4,3-*e*][1,4]diazepin-8-one,
1-Ethyl-5-(3-hydroxy-phenyl)-3-methyl-6,7-dihydro-1*H*-pyrazolo[4,3-*e*][1,4]diazepin-8-one,
1-Ethyl-5-(2-hydroxy-phenyl)-3-methyl-6,7-dihydro-1*H*-pyrazolo[4,3-*e*][1,4]diazepin-8-one,
1-Ethyl-3-methyl-5-(2,3,4-trimethoxy-phenyl)-6,7-dihydro-1*H*-pyrazolo[4,3-*e*][1,4]diazepin-8-one,
1-Ethyl-3,5-diphenyl-6,7-dihydro-1*H*-pyrazolo[4,3-*e*][1,4]diazepin-8-one,
1-Ethyl-5-(4-hydroxy-phenyl)-3-isopropyl-6,7-dihydro-1*H*-pyrazolo[4,3-*e*][1,4]diazepin-8-one,
5-(4-Hydroxy-phenyl)-3-isopropyl-1-propyl-6,7-dihydro-1*H*-pyrazolo[4,3-*e*][1,4]diazepin-8-one,
3-*tert*-Butyl-1-ethyl-5-(4-hydroxy-phenyl)-6,7-dihydro-1*H*-pyrazolo[4,3-*e*][1,4]diazepin-8-one,
5-(2,6-Dimethoxy-phenyl)-1-ethyl-3-methyl-6,7-dihydro-1*H*-pyrazolo[4,3-*e*][1,4]diazepin-8-one,
3-Ethoxymethyl-1-ethyl-5-phenyl-6,7-dihydro-1*H*-pyrazolo[4,3-*e*][1,4]diazepin-8-one

Compounds like substituted pyrazolo-[4,3-e]diazepines are preferred and more particularly, the following compounds :
1-Ethyl-3-isopropyl-5-phenyl-6,7-dihydro-1*H*-pyrazolo[4,3-*e*][1,4]diazepin-8-one,
3-*tert*-Butyl-1-ethyl-5-phenyl-6,7-dihydro-1*H*-pyrazolo[4,3*-e*][1,4]diazepin-8-one,
1-Ethyl-3-isopropyl-5-phenyl-6,7-dihydro-1*H*-pyrazolo[4,3-*e*][1,4]diazepin-8-thione,
5-(4-Amino-phenyl)-1-ethyl-3-isopropyl-6,7-dihydro-1*H*-pyrazolo[4,3-*e*][1 ,4]diazepin-8-one,
1-Ethyl-5-(4-hydroxy-phenyl)-3-methyl-6,7-dihydro-1*H*-pyrazolo[4,3-*e*][1 ,4]diazepin-8-one,
4-(1-Ethyl-3-isopropyl-8-oxo-1,6,7,8-tetrahydro-pyrazolo[4,3*-e*][1,4]diazepin-5-yl)-benzonitrile,
5-*tert*-Butyl-3-isopropyl-1-propyl-6,7-dihydro-1*H*-pyrazolo[4,3*-e*][1,4]diazepin-8-one,
4-(3-Isopropyl-8-oxo-1-propyl-1,6,7,8-tetrahydro-pyrazolo[4,3-*e*][1,4]diazepin-5-yl)-benzonitrile,
3-*tert*-Butyl-1-ethyl-5*-p*-tolyl-6,7-dihydro-1*H*-pyrazolo[4,3*-e*][1,4]diazepin-8-one,
3-Butyl-1-ethyl-5-phenyl-6,7-dihydro-1*H*-pyrazolo[4,3-*e*][1,4]diazepin-8-thione,
(±)3*-sec*-Butyl-1-ethyl-5-pyridin-4-yl-6,7-dihydro-1*H*-pyrazolo[4,3-*e*][1,4]diazepin-8-one,
(±)3*-sec*-Butyl-1-propyl-5-pyridin-4-yl-6,7-dihydro-1*H*-pyrazolo[4,3-*e*][1,4]diazepin-8-one,
3-Cyclohexyl-1-propyl-5-pyridin-4-yl-6,7-dihydro-1*H*-pyrazolo[4,3-*e*][1 ,4]diazepin-8-one,
3-*tert*-Butyl-5-(4-methoxy-phenyl)-1-propyl-6,7-dihydro-1*H*-pyrazolo[4,3-*e*][1,4]diazepin-8-one,
1-Ethyl-5-(2-hydroxy-phenyl)-3-methyl-6,7-dihydro-1*H*-pyrazolo[4,3-*e*][1,4]diazepin-8-one,
3-*tert*-Butyl-1-ethyl-5-(4-hydroxy-phenyl)-6,7-dihydro-1*H*-pyrazolo[4,3-*e*][1,4]diazepin-8-one,
1-Ethyl-3-isopropyl-5-phenyl-6,7-dihydro-1*H*-pyrazolo[4,3-*e*][1,4]diazepin-8-ylidène-cyanamide.

According to the use defined above, the medicament is under a form suitable for administration by systemic or local route.

The medicament may be adapted for inhalation and thus be presented under the form of a powder or a spray.

Systemic administration is preferred for treating diseases like multiple sclerosis, diabetes, allergic and auto-immune encephalomyelitis.

The PDE4 inhibitors utilized in the present invention include solvates, hydrates and pharmaceutically acceptable salts thereof.

Where it is appropriate to form a salt, the pharmaceutically acceptable salts include acetate, benzenesulfonate, benzoate, bitartrate, calcium acetate, camsylate, carbonate, citrate, edetate, edisylate, estolate, esylate, fumarate, gluceptate, gluconate, glutamate, glycoloylarsanilate, hexylresorcinate, hydrabamine, hydrobromide, hydrochloride, hydrogencarbonate, hydroxynaphthoate, iodide, isethionate, lactate, lactobionate, malate, maleate, mandelate, mesylate, methylnitrate, methylsulfate, mucate, napsylate, nitrate, pamoate (embonate), pantothenate, phosphate/diphosphate, polygalacturonate, salicylate, stearate, subacetate, succinate or hemi-succinate, sulfate or hemi-sulfate, tannate, tartrate or hemi-tartrate, theoclate, triethiodide, benzathine, chloroprocaine, choline, diethanolamine, ethylenediamine, meglumine, procaine, aluminium, ammonium, tetramethyl ammonium, calcium, lithium, magnesium, potassium, sodium, and zinc. (See also "Pharmaceutical salts" by Berge S.M. *et al.* (1997) J. Pharm. Sci. **66:** 1-19, which is incorporated herein by reference.)

Another object of the invention consists of a pharmaceutical composition for preventing or treating a disease associated with an excess in IL-12 production comprising an effective amount of at least one phosphodiesterase E4 (PDE4) inhibitor or a pharmaceutically acceptable salt thereof in combination with at least one pharmaceutically acceptable carrier.

The expression " an effective amount " of a PDE4 inhibitor according to the invention is intended to mean an amount of PDE4 inhibitor by dosage unit allowing the administration of from about 2 mg to about 1000 mg PDE4 inhibitor per day for an adult patient of normal weight (i.e. 70 kg).

Preferred PDE4 inhibitors to be used in a pharmaceutical composition of the invention are those defined above in the specification.

A pharmaceutical composition according to the invention may comprise a combination of two or more PDE4 inhibitors, preferably from 2 to 4 and most preferably from 2 to 3 PDE4 inhibitors.

The pharmaceutical composition according to the invention is under a form suitable for administration by systemic or local route.

Pharmaceutical compositions of a PDE4 inhibitor of the present invention, including one of its salts, are produced by formulating this active component in dosage unit form with at least one pharmaceutically acceptable carrier or excipient. For preparing pharmaceutical compositions from a PDE4 inhibitor used in this invention, inert, pharmaceutically acceptable carriers can be either solid or liquid.

Solid dosage forms for oral administration include capsules, tablets, pills, powders, and granules. In such solid dosage forms, the PDE4 inhibitor is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, carboxymethylcellulose, alginates, gelatin, polyvinylpyrrolidone, sucrose, and acacia, (c) humectants, as for example, glycerol, (d) disintegrating agents, as for example, agar-agar, calcium carbonate, potato or tapioca starch, alginic acid, certain complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid compositions of a similar type may also be employed as fillers in soft and hard-filled gelatin capsules using such excipients as lactose as well as high molecular weight polyethyleneglycols, and the like.

Solid dosage forms such as tablets, dragees, capsules, pills, and granules can be prepared with coatings and shells, such as enteric coatings and others well known in the art. They can also be of such composition that they release the PDE4 inhibitor, in a certain part of the intestinal tract in a delayed manner. Examples of embedding compositions which can be used are polymeric substances and waxes. The active component can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to a PDE4 inhibitor, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, and the like.

Suspensions, in addition to a PDE4 inhibitor, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administrations are preferably suppositories which can be prepared by mixing the compound of the present invention with suitable non-irritating excipients or carriers such as cocoa butter, polyethyleneglycol or a suppository wax, which are solid at ordinary temperatures but liquid at body temperature, and therefore melt in the rectum and release the active component.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable liquid carriers, diluents, solvents or vehicles include water, ethanol, polyols (propyleneglycol, polyethyleneglycol, glycerol, and the like), and suitable mixtures thereof.

These compositions may also contain adjuvants such as preserving, wetting, emulsifying, and dispersing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like.

Preferably the pharmaceutical preparation is in unit dosage form. In such form, the preparation is divided into unit doses containing appropriate quantities of a PDE4 inhibitor. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms. Some examples of dosage unit forms are tablets, capsules, pills, powders, suppositories, aqueous and nonaqueous oral solutions and suspensions, and parenteral solutions packaged in containers containing either one or some larger number of dosage units and capable of being subdivided into individual doses.

The percentage of the active component in the foregoing compositions can be varied within wide limits, but for practical purposes it is preferably present in a concentration of at least 10 % in a solid composition and at least 2 % in a primary liquid composition. The most satisfactory compositions are those in which a much higher proportion of the active component is present, for example, from 10 % to 90 % by weight.
This invention also pertains to a method for preventing and/or treating a disease associated with an excess in IL-12 production comprising administering to a subject in need of treatment an effective amount of at least one PDE4 inhibitor or a pharmaceutically acceptable salt thereof. Indeed the results of experiments are showing that:
- PDE4 inhibitors have a direct effect on either immature or maturing DC by inhibiting the production of IL-12p70 and other inflammatory cytokines such as TNF-α and to some extend IL- 6
- PDE4 inhibitors do not modify otherwise the phenotype of these cells.
- By the direct inhibitory effect of the PDE4 inhibitors on the cytokine production by DC, the balance of the Th-1 versus Th-2 differentiation of naive T cells is shifted in the direction of Th-2 to the detriment of Th-1 cells.

The term "patient " is intended to include a mammal, especially a human.

All that is required to practice the method of preventing and treating a disease associated with an excess in IL-12 production according to the present invention is to administer at least one PDE4 inhibitor in an amount that is effective to prevent or treat the damaged condition. The effective amount of PDE4 inhibitor to be utilized will generally be from about 2 mg to about 1000 mg per day for an adult patient of normal weight (i.e. 70 kg).

The invention is further illustrated, without being limited to the examples below.

### EXAMPLES

### MATERIAL AND METHODS OF THE EXAMPLES

### Materials and Methods

### Cytokines and Reagents

The PDE4 inhibitors Rolipram, Diazepino-Indole, Pirazolo [4,3-e]diazepine and Ariflo are kept as 0.1 M stock solutions in DMSO, stored at -20°C, and diluted into complete medium just before use. A 1:10⁴ final dilution of DMSO is included as a vehicle control in all experiments.

Human rGM-CSF (specific activity (SA) 1.5 x 10⁷ U/mg), human rIL-4 (S. A. 2.9 x 10⁷ U/mg), human rIFN-γ (S.A. 2 x 10⁷ U/mg) all obtained from R&D systems Europe (Oxon, U.K.).

### In vitro generation of dendritic cells (DC) from peripheral blood monocytes

Venous blood from healthy donors is collected by venipuncture in sodium-heparin containing tubes (VT100H; Venoject, Terumo Europe, Leuven, Belgium). Peripheral blood mononuclear cells (PBMC) are isolated by density centrifugation on Lymphoprep (Nycomed, Torshov, Norway). Subsequently, PBMC are centrifuged on a Percoll (Pharmacia, Uppsala, Sweden) gradient consisting of three density layers (1.076, 1.059 and 1.045 g/ml). The light density fraction floating on the middle layer, which contained predominantly monocytes, is seeded in 24-well culture plates (Costar, Cambridge, MA) at a density of 5x10⁵ cells/well. After 30 min of incubation at 37°C, nonadherent cells are removed; adherent cells are cultured in Iscove's modified Dulbecco's medium (Life Technologies, Paisley, U.K.) containing gentamycin (86 µg/ml; Duchefa, Haarlem, The Netherlands) and 1 % FCS (Hyclone, Logan, UT) and supplemented with GM-CSF (500 U/ml) and IL-4 (250 U/ml) to obtain DC. At day 3, the media, including the supplements, are refreshed. At day 6, immature CD1a⁺CD14⁻ DC are obtained. In some experiments, monocytes are purified from PBMC by negative selection using a monocyte isolation kit (Miltenyi Biotec, Bergisch Gladbach, Germany). The obtained monocyte fraction is then processed for in vitro culture in RPMI-1640 (Life Technologies, Paisley, U.K) containing 10 % FCS, 1% nonessential amino acids, 1% sodium pyruvate, 100 µg/ml kanamycine (all from Life Technologies, Paisley, U.K.) and supplemented with rhGM-CSF (500 U/ml) and rh-IL4 (250 U/ml) from R&D Systems Europe, Oxon, U.K.. At day 3, the media, including the supplements, were refreshed. In both protocols, similar preparation of immature CD1a⁺CD14⁻ DC are obtained.

### Induction of maturation of DC in the presence or absence of PDE4 inhibitors

At day 6, the maturation of CD1a⁺ DC was induced by a 2-day exposure to either LPS (250 ng/ml; Difco, Detroit, Ml) or a combination of the cytokines rhlL-1β (10 ng/ml), and TNF-α (25 ng/ml) both obtained from Pharma Biotechnologie, Hannover, Germany. Maturation was induced in the absence or presence of PDE4 inhibitors (10µM, unless stated otherwise) or PGE₂ (10⁻⁶ M; Sigma, St. Louis,MA). Where indicated, DC were kept in non-maturing conditions during 2 days.

### Induction of cytokine secretion by matured DC

At day 8, mature DC were harvested, washed extensively, and 2x10⁴ cells/200µl were stimulated with CD40L-transfected J558 cell line (2x10⁴ cells/well) in the absence or presence of IFN-γ (10³ U/ml) in 96-well flat bottom culture plates (Costar, Cambridge, MA) in IMDM containing 10% FCS. Supernatants were harvested after 24 h and stored at -20°C for determination of IL-12p70, IL-6, and TNF-α levels by ELISA.

### Analysis of expression of cell surface molecules by flow cytometry

The mouse mAbs against the following human molecules were used: CD1a (OKT6; Ortho Diagnostic System, Beerse, Belgium), ICAM-1, CD86 (Pharmingen, San Diego, CA), CD83 (HB15a, IgG2b; Immunotech, Marseille, France), followed by FITC-conjugated goat anti-mouse mAb (Jackson Immunoresearch Laboratories, West Grove, PA).

### Cytokine measurements

Measurements of IL-12p70 levels in culture supernatants were performed by a specific solid-phase sandwich ELISA OptEIA™ human IL-12P70 (Pharmingen San Diego, CA). Measurements of IL-6 and TNF-α were performed by ELISA using pairs of specific mAbs and recombinant cytokine standards obtained from BioSource International (Camarillo, CA). The limits of detection of these ELISA are as follows: IL-6, 20 pg/ml; IL-12p70, 3 pg/ml; and TNF-α, 20 pg/ml.

### Isolation of CD4⁺ CD45RA⁺ CD45RO⁻ naive T cells, cocultures with DC, and induction of memory-type cytokines in maturing Th cells

Naive Th cells (ThN) were isolated from peripheral blood leukocytes with the negative selection human CD4⁺/CD45RO⁻ column kit (R&D Systems Europe, Oxon, U.K.). This method yielded highly purified (>98%) CD4⁺ CD45RA⁺ CD45RO⁻ ThN as assessed by flow cytometry. DC that had been matured with rhIL-1β and TNFα, as described in the paragraph *Induction of maturation* of *DC in the presence of PDE4 inhibitors,* were extensively washed to remove any residual inhibitors. ThN (20.000 cells/well) were co-cultured in 96-well flat-bottom culture plates with 10.000 DC/well in the presence of 1 ng/ml SEB. On day 5, IL-2 (10U/ml) was added and the cultures were further expanded for another 9 days. On day 14, quiescent memory Th cells were harvested, washed, and re-stimulated for 6 h with 10 ng/ml PMA, 1 µg/ml ionomycin and 10 µg/ml Brefeldin A (all obtained from Sigma). Cells were fixed with 4%paraformaldehyde, permeabilized with PBS containing BSA (0.5%) and saponin (0.5%), stained with FITC-Iabeled anti-IFN-α and PE-labeled anti-IL-4 mAb (Becton & Dickinson), and analyzed on a FACScan (Becton & Dickinson).

The experiments were performed according to the techniques described in the Materials and Methods Section above.

### EXAMPLE 1 : Direct effect of PDE4 inhibitors on cytokine secretion by immature Dendritic cells (DC).

Production of IL-12 by DC can be induced by factors such as LPS togethe with, IFN-γ or by the ligation of CD40 after contact with CD40L-expressing T cells

As shown in Fig. 1, immature DC stimulated with LPS and IFN-γ in the presence of Rolipram or Ariflo showed reduced ability to produce IL-12p70 and TNF-α in comparison to DMSO-treated DC. In contrast, the production of IL-6 by the immature DC was not affected by any of the inhibitors.

Accordingly, this example demonstrates that PDE4 inhibitors have a direc effect on dendritic cells by reducing significantly the secretion of their cytokines.

### EXAMPLE 2 : Comparison of direct effect of several PDE4 inhibitors on IL-12p70 secretion by immature dendritic cells.

IL-12 production by dendritic cells was stimulated with J558-CD40L cells in the absence of any PDE inhibitor or in the presence of 10 µM of PDE4 inhibitors (Ariflo, Rolipram, Diazepino-indole or Pyrazolo [4,3-e] diazepine). The data presented in Figure 2 show that all the PDE4 inhibitors tested were able to inhibit IL-12 production by stimulated dendritic cells with an inhibitory activity of the same order of magnitude.

### EXAMPLE 3 : Presence of PDE4 inhibitors during maturation of DC does no alter the mature phenotype of DC

The maturation process of DC is associated with the appearance of CD83 and upregulation of co-stimulatory molecules like CD86. The study whether the presence of PDE4 inhibitors during DC maturation could affect the cell surface expression of the DC-associated marker CD1a, the co-stimulatory molecules CD86 and ICAM-1, and the DC maturation marker CD83, therefore the study o the expression of these markers after treatment has been performed.

As shown in Figures 3 A and B, PDE4 inhibitor treatment of DC for 2 day did not affect the expression of surface molecules induced by the maturatio factors (TNF-α plus IL-1β, referred to as MF) or by LPS when compared t vehicle-treated control mature DC.

The data thus indicate that although PDE4 inhibitors modulated th cytokine production of DC, they did not prevent their phenotypic maturatio induced by inflammatory cytokines or LPS.

### EXAMPLE 4 : Effect of PDE4 inhibitor treatment during maturation of DC on their subsequent cytokine secretion

The effect of treatment of DC with PDE4 inhibitor only during their in vitro maturation on their ability to produce cytokines upon subsequent stimulation wa studied. PGE2 was used as a control. DC maturation was induced by a combination of inflammatory cytokines (TNF-α plus IL-1β) in the absence or ii the presence of PDE4 inhibitors.

As shown in Figure 4, DC matured with IL-1β plus TNF-α in the additiona presence of PDE4 inhibitors showed a reduced capacity to produce IL-12p70 and TNF-α (between 40-60% inhibition) upon subsequent stimulation by J558 transfected CD40L cells.

From the experiment shown, DC matured in the presence of PDE4 inhibitors showed inhibited (as shown in Fig. 4), IL-6 production.

### EXAMPLE 5 : Effect of PDE4 inhibitors on naive T cell differentiation.

The experiments were performed according to the techniques described in the Materials and Methods Section above.

Naive Th cells (ThN) were isolated from human peripheral blood leukocytes and then co-cultured with dendritic cells matured in the presence of various PDE4 inhibitors . The differentiation of the naive Th cells in respectively Th1 and Th2 cells were visualised by intracellular staining of the T-cells with labelled anti-IFN-γ and anti-IL-4 monoclonal antibodies and flow cytometry analysis.

PDE4 inhibitors were added during dendritic cell (DC) maturation in the presence of IL1β and TNFα (maturation factors or MF). After two days of maturation, the dendritic cells were washed and used to prime naive T cells.

Differentiation towards Thr/Th2 was evaluated by intracellular staining of IFNγ positive and IL4 positive cells respectively and expressed as percentages of positive cells.

The results are reported in table 1 hereunder.

**Table 1**

| | **Experiment 1** | | **Experiment 2** | | **Experiment 3** | |
|---|---|---|---|---|---|---|
| | IFN-γ (%) | IL-40(%) | IFN-γ (%) | IL-4 (%) | IFN-g (%) | IL-4 (%) |
| MF-DC-DMSO | 14.1 | 12.6 | 42.5 | 0.9 | 37.7 | 1.7 |
| MF-DC-Ariflo(10µM) | 4.7 | 15.1 | 29.6 | 2.0 | 24.0 | 6.2 |
| MF-DC-Rolipram (10µM) | 7.2 | 13.5 | 35.4 | 1.1 | 24.5 | 5.7 |

As seen in table 1, the maturation of dendritic cells in the presence of PDE4 inhibitors promoted the differentiation of naive T cells towards a Th2-like phenotype as shown by intracellular IFNγ/IL4 staining.

More precisely, the direct activity of PDE4 inhibitors on dendritic cells induces a decrease of INF-γ production in the Th1 population.

These data indicate that PDE4 inhibitors modulate the dendritic cell polarizing capacity and thus that PDE4 inhibitors are highly useful for the treatment of any disease involving IL-12 production by dendritic cells and wherein an indesirable number of Th1-type T cells are produced.

## Claims

1. The use of at least one PDE4 inhibitor or a pharmaceutically acceptable salt thereof for manufacturing a medicament for preventing and/or treating a disease associated with an excess in IL-12 production.

2. The use according to claim 1, wherein the PDE4 inhibitor is selected from the group consisting of Rolipram, Ariflo, Diazepino-indole compounds preferably [1,4]diazepino[6,7,1-*hi*] indoles, 1-aminotriazolo[4,3-a]quinazolin-5-ones and/or -5-thiones compounds, substituted pyrazolo-[4,3-e]diazepines and their metobolites.

3. The use according to any one of claims 1 and 2, wherein the disease is selected from the group consisting of Inflammatory of the bronchi/pathologies affecting the bronchus such as Bronchoconstriction, multiple organ failure, Osteoarthritis, Septic shock (septicaemia), Inflammatory complaints or disorders, (Chronic) inflammatory diseases of the digestive system including intestine (hemorrhagic rectocolitis, ulcerative colitis, IBD) with auto-immune component, chronic lymphatic leukaemia, Hepatic failure including following immunologic or inflammatory liver injury, Graft rejection, Crohn's disease, Chronic obstructive bronchopneumopathy (chronic bronchitis, emphysema, COPD), acute pulmonary attack, ischemia-induced neuronal damages, Reperfusion ischaemia diseases related to a high level of TNF-α, acute respiratory distress syndrome, acute pancreatitis, auto-immune demyelinating disorders, multiple sclerosis, Type I diabetes, chronic/relapsing allergic encephalomyelitis, auto-immune encephalomyelitis, inflammatory colitis, arthritis including rheumatoid arthritis, sarcoidiosis, hypersensitivity pneumonitis, auto-immune uveitis, Inflammatory Bowel Disease (IBD), psoriasis, eczema, contact dermatitis, as well as other Th1-mediated human diseases.

4. The use according to any one of claims 1 to 3 wherein the medicament is under a form suitable for administration by systemic or local route.

5. A pharmaceutical composition for preventing and/or treating a disease associated with an excess in IL-12 production comprising an effective amount of at least one phosphodiesterase E4 (PDE4) inhibitor or a pharmaceutically acceptable salt thereof in combination with at least one pharmaceutically acceptable carrier.

6. The pharmaceutical composition according to claim 5 wherein the PDE4 inhibitor is selected from the group consisting of Rolipram, Ariflo, Diazepino-indole compounds and preferably [1,4]diazepino[6,7,1-*hi*] indoles, 1-aminotriazolo[4,3-a]quinazolin-5-ones and/or -5-thiones compounds, substituted pyrazolo-[4,3-*e*]diazepines compounds and their metabolites.

7. The pharmaceutical composition according to any one of claims 5 or 6, for preventing or curing diseases selected from the group consisting of Inflammatory of the bronchi/pathologies affecting the bronchus such as Bronchoconstriction, multiple organ failure, Osteoarthritis, Septic shock (septicaemia), Inflammatory complaints or disorders, (Chronic) inflammatory diseases of the digestive system including intestine (hemorrhagic rectocolitis, ulcerative colitis, IBD) with auto-immune component, chronic lymphatic leukaemia, Hepatic failure including following immunologic or inflammatory liver injury, Graft rejection, Crohn's disease, Chronic obstructive bronchopneumopathy (chronic bronchitis, emphysema, COPD), acute pulmonary attack, ischemia-induced neuronal damages, Reperfusion ischaemia diseases related to a high level of TNF-α, acute respiratory distress syndrome, acute pancreatitis, auto-immune demyelinating disorders, multiple sclerosis, Type I diabetes, chronic/relapsing allergic encephalomyelitis, auto-immune encephalomyelitis, inflammatory colitis, arthritis including rheumatoid arthritis, sarcoidiosis, hypersensitivity pneumonitis, auto-immune uveitis, Inflammatory Bowel Disease (IBD), psoriasis, eczema, contact dermatitis, as well as other Th1-mediated human diseases

8. The pharmaceutical composition according to any one of claims 5 to 7 which is under a form suitable for administration by systemic or local route.
